(19) Europäisches Patentamt
European Patent Office
Office européen des brevets

(11) EP 2 346 602 B1

(12) **EUROPÄISCHE PATENTSCHRIFT**

(45) Veröffentlichungstag und Bekanntmachung des Hinweises auf die Patenterteilung:
**19.03.2014 Patentblatt 2014/12**

(21) Anmeldenummer: **09782817.2**

(22) Anmeldetag: **09.09.2009**

(51) Int Cl.:
***B01J 21/04*** *(2006.01)* ***B01J 23/72*** *(2006.01)*
***C07C 209/16*** *(2006.01)* ***C07C 209/26*** *(2006.01)*
***C07C 211/08*** *(2006.01)* ***C07C 211/10*** *(2006.01)*
***C07C 211/12*** *(2006.01)* ***C07C 211/14*** *(2006.01)*
***C07C 211/35*** *(2006.01)* ***B01J 35/02*** *(2006.01)*
***B01J 37/00*** *(2006.01)* ***B01J 37/18*** *(2006.01)*
***B01J 37/02*** *(2006.01)*

(86) Internationale Anmeldenummer:
**PCT/EP2009/061691**

(87) Internationale Veröffentlichungsnummer:
**WO 2010/031719 (25.03.2010 Gazette 2010/12)**

(54) **VERFAHREN ZUR KONTINUIERLICHEN HERSTELLUNG EINES AMINS UNTER VERWENDUNG EINES ALUMINIUM-KUPFER-KATALYSATORS**

METHOD FOR THE CONTINUOUS PRODUCTION OF AN AMINE USING AN ALUMINIUM-COPPER CATALYST

PROCÉDÉ DE PRÉPARATION EN CONTINU D'UNE AMINE AU MOYEN D'UN CATALYSEUR À BASE D'ALUMINIUM ET DE CUIVRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **19.09.2008 EP 08164749**

(43) Veröffentlichungstag der Anmeldung:
**27.07.2011 Patentblatt 2011/30**

(73) Patentinhaber: **BASF SE**
**67056 Ludwigshafen (DE)**

(72) Erfinder:
- **ERNST, Martin**
  **69115 Heidelberg (DE)**
- **STEIN, Bernd**
  **64665 Alsbach-Hähnlein (DE)**
- **MAAS, Steffen**
  **55270 Bubenheim (DE)**
- **PASTRE, Jörg**
  **64625 Bensheim (DE)**
- **JOHANN, Thorsten**
  **67063 Ludwigshafen (DE)**
- **MELDER, Johann-Peter**
  **67459 Böhl-Iggelheim (DE)**

(56) Entgegenhaltungen:
**EP-A1- 0 235 651** **EP-A2- 0 514 692**
**WO-A1-2005/110969** **WO-A1-2007/036499**
**DE-A1- 2 445 303** **DE-A1- 19 859 776**
**US-A- 4 910 304**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 346 602 B1

## Beschreibung

Die vorliegende Erfindung betrifft ein Verfahren zur kontinuierlichen Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300°C in Gegenwart eines kupfer- und aluminiumoxidhaltigen Katalysators.

Die Verfahrensprodukte finden u.a. Verwendung als Zwischenprodukte bei der Herstellung von Kraftstoffadditiven (US 3,275,554 A; DE 21 25 039 A und DE 36 11 230 A), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

EP 257 443 A (BASF AG) betrifft ein Verfahren zur Herstellung von Trialkylaminen (z. B. Dimethylethylamin) durch Umsetzung von Ammoniak mit Alkoholen in Gegenwart von Alkalihydroxid in der Flüssigphase an einem im wesentlichen nur Kupfer enthaltenden Katalysator.

EP 227 904 A (BASF AG) lehrt die Herstellung von Dimethylethylamin oder N,N-Dimethylcyclohexylamin in der Flüssigphase durch Umsetzung von Dimethylamin mit Cyclohexanol in Gegenwart von Alkalihydroxid und eines Katalysators, der als aktives Metall im wesentlichen nur Kupfer enthält bzw. ein reiner Kupferkatalysator ist.

US 4,910,304 A (BASF AG) offenbart die Herstellung von N-Methylpiperidin und N-Methylmorpholin durch Reaktion von Pentandiol bzw. Diethylenglykol (DEG) mit Methylamin und 45 %iger wässriger KOH-Lösung an einem Cu/Al-Vollkontakt bei 245°C und 250 bar.

EP 137 478 A (BASF AG) betrifft Verfahren zur Herstellung von N-Methylpiperidin oder N-Methylmorpholin durch katalytische Aminierung von Pentandiol mit Methylamin in der Gasphase zwischen 5 und 25 bar an einem kupferhaltigen Katalysator, der durch Temperung eines basischen Kupfer und Aluminium enthaltenden Carbonats erhalten wurde.

EP 235 651 A1 (BASF AG) lehrt ein Verfahren zur Herstellung von N-Methylpiperazin aus Diethanolamin und Methylamin an metallhaltigen Katalysatoren. Die Umsetzung wird in der Flüssigphase (Rieselfahrweise) durchgeführt (Seite 3, letzter Absatz). Gemäß Beispiel wird ein $Cu/Al_2O_3$-Katalysator in Tablettenform, Höhe = Durchmesser = 4 mm, eingesetzt.

EP 816 350 A (BASF AG) beschreibt Verfahren zur Herstellung von N-Methylpiperidin und N-Methylmorpholin durch Umsetzung von primärem Amin mit einem Diol an einem Kupferkatalysator, welcher durch Tränkung von $SiO_2$-Kugeln mit basischem Kupfercarbonat erhalten wurde, in der Flüssig- oder Gasphase.

US 4,739,051 A (BASF AG) lehrt die Herstellung von Morpholin und Piperidin durch Reaktion von DEG oder Pentandiol mit Ammoniak unter Hydrierbedingungen in der Gasphase bei Normaldruck und 200°C an einem Cu/Ni/Al-Vollkatalysator mit Ausbeuten von 97 bzw. 95 %.

EP 514 692 A2 (BASF AG) offenbart Verfahren zur Herstellung von Aminen aus Alkoholen in Gegenwart Kupfer und Nickel und Zirkonium- und/oder Aluminiumoxid enthaltender Katalysatoren.

EP 1 020 455 A (BASF AG) betrifft ein Verfahren zur Herstellung von 2,2'-Dimorpholinodiethylether durch Umsetzung von Diethylenglykol (DEG) mit Ammoniak unter Druck und bei erhöhter Temperatur in Gegenwart von Wasserstoff und einem kupferhaltigen Hydrierkatalysator.

EP 1 106 600 A (BASF AG) lehrt die Verwendung von $ZrO_2$-Cu-Co-Ni - Katalysatoren in Aminierungsreaktionen. Gemäß Beispiel werden 5 x 3 mm Tabletten als Katalysatorformkörper eingesetzt.

US 4,806,690 A (Dow Chemical Comp.) betrifft die Aminierung von Alkoholen, Aledhyden und Ketonen in Gegenwart eines Co-Cu-Fe und Zn und/oder Zr- Katalysators. In einem Beispiel werden 8-16 mesh Katalysator-Partikel verwendet.

DE 19 85 9776 A (BASF AG) betrifft die Herstellung von Aminen durch Umsetzung von Alkoholen oder Aldehyden bzw. Ketonen mit Aminen an einem Katalysator aus Kupfer und $TiO_2$, welchem vor der Verformung des Katalysatormaterials metallisches Kupfer zugesetzt wurde. Gemäß Beispiel wird der Katalysator als Tabletten-Formkörper mit einem Durchmesser von 3 mm eingesetzt.

EP 440 829 A1 (US 4,910,304) (BASF AG) beschreibt die Aminierung von Diolen an Kupferkatalysatoren. Die Umsetzung wird in der Flüssigphase (Rieselfahrweise) durchgeführt (Seite 3, letzter Absatz). Geeignete Katalysatoren sind die in DE 24 45 303 A (BASF AG) offenbarten Katalysatoren, die durch Temperung eines basischen Kupfer und Aluminium enthaltenen Carbonats der allgemeinen Zusammensetzung $Cu_mAl_6(CO_3)_{0,5m}O_3(OH)_{m+12}$, wobei m einen beliebigen, auch nicht ganzzahligen, Wert zwischen 2 und 6 bedeutet, erhältlich sind, beispielsweise der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird.

In den Beispielen der EP 440 829 A wird der Katalysator als Zylinder-Formkörper mit 3 mm Länge und 3 mm Durchmesser eingesetzt.

WO 07/036496 A1 (BASF AG) beschreibt die Umsetzung von Diethylenglykol mit Ammoniak in Gegenwart von

Cu-Ni-Co - Katalysatoren. Der Katalysator-Formkörper weist bei Tablettenform eine Höhe von < 3 mm auf.

WO 07/036498 A1 (BASF AG) betrifft die Umsetzung von Monoethanolamin mit Ammoniak in Gegenwart von Cu-Ni-Co - Katalysatoren. Der Katalysator-Formkörper weist bei Tablettenform eine Höhe von < 3 mm auf.

WO 07/093514 A1 und WO 07/093552 A1 (beide BASF AG) lehren die Umsetzung von Monoethylenglykol mit Ammoniak in Gegenwart von Ru-Co - Katalysatoren. Der Katalysator-Formkörper weist bei Tablettenform eine Höhe von < 3 mm auf.

WO 05/110969 A1 (BASF AG) beschreibt ein Verfahren zur kontinuierlichen Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300°C in Gegenwart eines kupferhaltigen Katalysators, wobei die katalytisch aktive Masse des Katalysators vor dessen Reduktion mit Wasserstoff 20 bis 85 Gew.-% Aluminiumoxid ($Al_2O_3$), Zirkoniumdioxid ($ZrO_2$), Titandioxid ($TiO_2$) und/oder Siliziumdioxid ($SiO_2$) enthält und die Umsetzung in der Gasphase isotherm in einem Rohrreaktor erfolgt.

Der vorliegende Erfindung lag die Aufgabe zugrunde, ein verbessertes wirtschaftliches Verfahren zur Herstellung eines Amins aufzufinden. Insbesondere soll das Verfahren bessere Ausbeuten, Raum-Zeit-Ausbeuten (RZA) und Selektivitäten ermöglichen.

**[Raum-Zeit-Ausbeuten werden angegeben in ‚Produktmenge / (Katalysatorvolumen • Zeit)' (kg/($l_{Kat.}$ • h)) und/oder ‚Produktmenge / (Reaktorvolumen • Zeit)' (kg/($l_{Reaktor}$ • h)].**

Demgemäß wurde ein Verfahren zur kontinuierlichen Herstellung eines Amins durch Umsetzung eines primären oder sekundären Alkohols mit aliphatischer OH-Funktion, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300°C in Gegenwart eines kupfer- und aluminiumoxidhaltigen Katalysators gefunden, welches dadurch gekennzeichnet ist, dass die Umsetzung in der Gasphase erfolgt und die katalytisch aktive Masse des Katalysators vor dessen Reduktion mit Wasserstoff

20 bis 75 Gew.-% Aluminiumoxid,
20 bis 75 Gew.-% sauerstoffhaltige Verbindungen des Kupfers, berechnet als CuO.
0 bis 2 Gew.-% sauerstoffhaltige Verbindungen des Natriums, berechnet als $Na_2O$, und weniger als 1 Gew.-% sauerstoffhaltige Verbindungen des Nickels, berechnet als NiO, enthält
und der Katalysatorformkörper eine Tablettenform, mit einem Durchmesser im Bereich von 1,1 bis 3,5 mm und einer Höhe im Bereich von 1,1 bis 3,5 mm, aufweist.

Erfindungsgemäß wurde u.a. die vorteilhafte Kombination des spezifischen Katalysators mit einer spezifischen Katalysatorformkörper-Dimension, mit einer bevorzugt isothermen Fahrweise (Aminierung des/der genannten Edukte), in der Gasphasen-Aminierung erkannt.

Mit dem erfindungsgemäßen Verfahren werden z. B. gegenüber 5 x 5 mm Tabletten höhere Raum-Zeit-Ausbeuten erzielt und die Reaktions-Selektivität ist verbessert, u.a. deshalb, weil weniger Scramblingprodukte auftreten, d. h. Nebenprodukte, die dadurch entstehen, dass Alkylgruppen intra- oder intermolekular übertragen werden (z. B. Disproportionierung von Alkylamin, z. B. DMA zu TMA, MMA, DMA; vgl. Beispiel 1).

Überraschenderweise wird darüber hinaus im erfindungsgemäßen Verfahren z .B. gegenüber 5 x 5 mm Tabletten eine größere Katalysatoraktivität, d. h. eine höhere Katalysatorbelastung bei zumindest gleichen Ausbeuten, erreicht und auch die Reaktortemperatur kann bei zumindest gleichen Ausbeuten vorteilhaft vergleichsweise niedriger eingestellt werden.

Insgesamt wird durch die Verkleinerung der Katalysatorgeometrie im erfindungsgemäßen Verfahren eine deutliche Effizienzsteigerung bei der Produktion von Aminierungsprodukten, ausgehend von Aldehyden, Ketonen und Alkoholen in der Gasphase erreicht.

Der Katalysatorformkörper weist eine Tablettenform, mit einem Durchmesser im Bereich von 1,1 bis 3,5 mm und einer Höhe im Bereich von 1,1 bis 3,5 mm, auf.

Der Katalysatorformkörper weist besonders bevorzugt eine Tablettenform, mit einem Durchmesser im Bereich von 1,2 bis 3,2 mm, insbesondere 1,3 bis 2,8 mm, weiter besonders 1,4 bis 2,5 mm, und einer Höhe im Bereich von 1,2 bis 3,2 mm, insbesondere 1,3 bis 2,8 mm, weiter besonders 1,4 bis 2,5 mm, auf.

Ganz besonders bevorzugt liegt im tablettenförmigen Katalysatorkörper das Verhältnis Durchmesser; Höhe im Bereich von 0,7 bis 2,0, insbesondere im Bereich von 0,8 bis 1,5, weiter besonders im Bereich von 0,9 bis 1,2.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial Aluminiumoxid ($Al_2O_3$) als zur katalytisch aktiven Masse gehörig gewertet.

Die Katalysatoren werden dergestalt eingesetzt, dass man die katalytisch aktive Masse nach Mahlung, Vermischung mit Formhilfsmitteln, Formung und Temperung als Katalysatorformkörper - nämlich als Tabletten - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und des o. g. Katalysatorträgermaterials definiert und enthält im wesentlichen die folgenden Bestandteile:

Aluminiumoxid ($Al_2O_3$) und sauerstoffhaltige Verbindungen des Kupfers und bevorzugt sauerstoffhaltige Verbindungen des Natriums.

Die Summe der o. g. Bestandteile der katalytisch aktiven Masse, berechnet als $Al_2O_3$, CuO und $Na_2O$, beträgt üblicherweise 70 bis 100 Gew.-%, bevorzugt 80 bis 100 Gew.-%, besonders bevorzugt 90 bis 100 Gew.-%, weiter bevorzugt 98 bis 100 Gew.-%, weiter bevorzugt $\geq$ 99 Gew.-%, ganz besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:

Übergangsmetalle, wie Ni bzw. NiO, Co bzw. CoO, Re bzw. Rheniumoxide, Mn bzw. $MnO_2$, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. $CeO_2$ oder Pr bzw. $Pr_2O_3$; Alkalimetalloxide, wie $K_2O$; Alkalimetallcarbonate, wie $Na_2CO_3$; Erdalkalimetalloxide, wie CaO, SrO; Erdalkalimetallcarbonate, wie $MgCO_3$, $CaCO_3$ und $BaCO_3$; Boroxid ($B_2O_3$).

Die katalytisch aktive Masse der im erfindungsgemäßen Verfahren eingesetzten Katalysatoren enthält nach deren letzter Wärmebehandlung und vor dessen Reduktion mit Wasserstoff

20 bis 75 Gew.-%, bevorzugt 25 bis 65 Gew.-%, besonders bevorzugt 30 bis 55 Gew.-%, Aluminiumoxid ($Al_2O_3$) und
20 bis 75 Gew.-%, bevorzugt 30 bis 70 Gew.-%. besonders bevorzugt 40 bis 65 Gew.-%, ganz besonders bevorzugt 45 bis 60 Gew.-%, sauerstoffhaltige Verbindungen des Kupfers, berechnet als CuO,
0 bis 2 Gew.-%, bevorzugt 0,05 bis 1 Gew.-%, besonders bevorzugt 0,1 bis 0,5 Gew.-%, sauerstoffhaltige Verbindungen des Natriums, berechnet als $Na_2O$,
weniger als 1 Gew.-%. z. B. 0,1 bis weniger als 1 Gew.-%. 0 bis 0,8 Gew.-%, sauerstoffhaltige Verbindungen des Nickels, berechnet als NiO.

Die katalytisch aktive Masse des Katalysators enthält vor dessen Reduktion mit Wasserstoff besonders bevorzugt weniger als 1 Gew.-%, z. B. 0 bis 0,5 Gew.-%, sauerstoffhaltige Verbindungen des Kobalts, berechnet als CoO.

Ganz besonders bevorzugt enthält die katalytisch aktive Masse des im erfindungsgemäßen Verfahren eingesetzten Katalysators kein Nickel, kein Kobalt und/oder kein Ruthenium, jeweils weder in metallischer (Oxidationsstufe 0) noch in einer ionischen, insb. oxidierten, Form.

Bei den sauerstoffhaltigen Verbindungen des Kupfers handelt es sich insbesondere um Kupfer-(I)-oxid und Kupfer-(II)-oxid, bevorzugt um Kupfer-(II)-oxid.

Ganz besonders bevorzugt enthält die katalytisch aktive Masse des im erfindungsgemaßen Verfahren eingesetzten Katalysators kein Zirkoniumdioxid ($ZrO_2$), Titandioxid ($TiO_2$) und/oder Siliziumdioxid ($SiO_2$).

In einer besonders bevorzugten Ausführungsform enthält die katalytisch aktive Masse der im erfindungsgemäßen Verfahren eingesetzten Katalysatoren keine weitere katalytisch aktive Komponente, weder in elementarer noch in ionischer Form.
in der besonders bevorzugten Ausführungsform ist die katalytisch aktive Masse nicht mit weiteren Metallen oder Metallverbindungen dotiert.
Bevorzugt sind jedoch aus der Metallgewinnung von Cu, ggf. Ni, herrührende übliche Begleit-Spurenelemente hiervon ausgenommen.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten Aluminium, Kupfer, ggf. Natrium mit Wasser und nachfolgendes Extrudieren und Tempern der so erhaltenen Masse erhältlich.

Die im erfindungsgemäßen Verfahren verwendeten Katalysatoren können auch durch Tränkung von Aluminiumoxid ($Al_2O_3$), das beispielsweise in Form von Pulver oder Tabletten-Formkörpem vorliegt, hergestellt werden.

Aluminiumoxid kann dabei in verschiedenen Modifikationen eingesetzt werden, bevorzugt sind $\alpha$- (alpha), $\gamma$-(gamma) oder $\theta$-$Al_2O_3$ (theta-$Al_2O_3$). Besonders bevorzugt wird $\gamma$-$Al_2O_3$ eingesetzt.

Die Herstellung von Formkörpern des Aluminiumoxids kann nach den üblichen Verfahren erfolgen.

Die Tränkung des Aluminiumoxids erfolgt ebenfalls nach den üblichen Verfahren, wie z. B. in EP 599 180 A, EP 673 918 A oder A. B. Stiles, Catalyst Manufacture - Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden. Die Masse wird im Anschluss an die Tränkung getrocknet und ggf. kalziniert.

Die Tränkung kann nach der sogenannten "incipient wetness"-Methode erfolgen, bei der das anorganische Oxid (d. h. Aluminiumoxid) entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu kalzinieren. Die mehrstufige Tränkung ist vorteilhaft besonders dann anzuwenden, wenn das anorganische Oxid mit einer größeren Metallmenge beaufschlagt werden soll.

Zur Aufbringung mehrerer Metallkomponenten auf das anorganische Oxid kann die Tränkung gleichzeitig mit ggf. allen Metallsalzen oder in beliebiger Reihenfolge der ggf. einzelnen Metallsalze nacheinander erfolgen.

Bevorzugt werden zur Herstellung der im erfindungsgemäßen Verfahren verwendeten Katalysatoren Fällungsmethoden angewandt. So können sie beispielsweise durch eine gemeinsame Fällung der Komponenten aus einer wässrigen Salzlösung mittels Mineralbasen in Gegenwart einer Aufschlämmung einer schwerlöslichen, sauerstoffhaltigen Aluminium-Verbindung und anschließendes Waschen, Trocknen und Calcinieren des erhaltenen Präzipitats erhalten werden. Als schwerlösliche, sauerstoffhaltige Aluminiumverbindung kann beispielsweise Aluminiumoxid Verwendung finden. Die Aufschlämmungen der schwerlöslichen Aluminiumverbindung kann durch Suspendieren feinkörniger Pulver dieser Verbindung in Wasser unter kräftigem Rühren hergestellt werden. Vorteilhaft werden diese Aufschlämmungen durch Ausfällen der schwerlöslichen Aluminiumverbindung aus wässrigen Aluminium-Salzlösungen mittels Mineralbasen erhalten.

Bevorzugt werden die im erfindungsgemäßen Verfahren verwendeten Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze des eingesetzten Metalls/der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden wie üblich zu den erfindungsgemäß verwendetenen Katalysatoren weiterverarbeitet. Nach dem Waschen werden sie im bevorzugt bei 80 bis 200°C, vorzugsweise bei 100 bis 150°C, getrocknet und danach calciniert. Die Calcinierung wird bevorzugt bei Temperaturen zwischen 300 und 800°C, vorzugsweise 400 bis 600°C, insbesondere 450 bis 550°C, ausgeführt.

Nach der Calcinierung wird der Katalysator zweckmäßigerweise konditioniert, sei es, dass man ihn durch Vermahlen auf eine bestimmte Korngröße einstellt und/oder dass man ihn nach seiner Vermahlung mit Formhilfsmitteln wie Graphit oder Stearinsäure vermischt, mittels einer Presse zu den Formlingen, nämlich Tabletten, verpresst und tempert. Die Tempertemperaturen entsprechen dabei bevorzugt den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren werden sie üblicherweise vorreduziert. Sie können jedoch auch ohne Vorreduktion eingesetzt

werden, wobei sie dann unter den Bedingungen der hydrierenden Aminierung durch den im Reaktor vorhandenen Wasserstoff reduziert werden.

Zur Vorreduktion werden die Katalysatoren zunächst bei bevorzugt 150 bis 200°C über einen Zeitraum von z. B. 12 bis 20 Stunden einer Stickstoff-Wasserstoff-Atmosphäre ausgesetzt und anschließend noch bis zu ca. 24 Stunden bei bevorzugt 200 bis 400°C in einer Wasserstoffatmosphäre behandelt. Bei dieser Vorreduktion wird ein Teil der in den Katalysatoren vorliegenden sauerstoffhaltigen Metallverbindung/en zu dem/den entsprechenden Metall/en reduziert, so dass diese gemeinsam mit den verschiedenartigen Sauerstoffverbindungen in der aktiven Form des Katalysators vorliegen.

Die Umsetzung gemäß erfindungsgemäßem Verfahren erfolgt bevorzugt in einem Rohrreaktor.

Die Umsetzung im Rohrreaktor gemäß dem erfindungsgemäßen Verfahren erfolgt ganz besonders bevorzugt in einer Kreisgasfahrweise.

Das Kreisgas, das bevorzugt zum überwiegenden Teil aus Wasserstoff besteht, dient zum einen der Verdampfung der Edukte und zum anderen als Reaktionspartner für die Aminierungsreaktion.

In der Kreisgasfahrweise werden die Ausgangsstoffe (Alkohol, Aldehyd und/oder Keton, Wasserstoff und die Stickstoffverbindung) bevorzugt in einem Kreisgasstrom verdampft und gasförmig dem Reaktor zugeführt.

Die Edukte (Alkohol, Aldehyd und/oder Keton, die Stickstoffverbindung) können auch als wässrige Lösungen verdampft und mit dem Kreisgasstrom auf das Katalysatorbett geleitet werden.

Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors". Ganz besonders bevorzugt erfolgt die Umsetzung in einem Rohrbündelreaktor oder in einer Monostranganlage.

Bei einer Monostranganlage besteht der Rohrreaktor, in dem die bevorzugt isotherme Umsetzung erfolgt, aus einer Hintereinanderschaltung mehrerer (z. B. zweier oder dreier) einzelner Rohrreaktoren.

Die Kreisgasmenge liegt bevorzugt im Bereich von 40 bis 1500 $m^3$ (bei Betriebsdruck) / [$m^3$ Katalysator (Schüttvolumen) • h], insbesondere im Bereich von 100 bis 700 $m^3$ (bei Betriebsdruck) / [$m^3$ Katalysator (Schüttvolumen) • h].

Das Kreisgas enthält bevorzugt mindestens 10, besonders 50 bis 100, ganz besonders 80 bis 100, Vol.-% $H_2$.

Die bevorzugt isotherme Umsetzung gemäß dem erfindungsgemäßen Verfahren erfolgt bevorzugt mit einer Temperaturabweichung von maximal +/- 8°C, besonders maximal +/- 5°C, insbesondere maximal +/- 4°C, ganz besonders maximal +/- 3°C, z. B. maximal +/- 0 bis +/- 2°C oder maximal +/- 0 bis +/- 1°C.

Diese Temperaturabweichungen beziehen sich auf die jeweiligen Temperaturen im jeweiligen Katalysatorbett, und zwar beim Eintritt der Edukte in das Katalysatorbett und beim Austritt der Reaktionsmischung aus dem Katalysatorbett.

Dabei können mehrere Katalysatorbetten parallel oder in Reihe ge- oder verschaltet sein.

Sind mehrere Katalysatorbetten in Reihe geschaltet, beziehen sich die genannten Temperaturabweichungen bei der erfindungsgemäß bevorzugten isothermen Fahrweise auf die jeweilige Temperatur im Katalysatorbett, und zwar beim Eintritt der Edukte in das erste Katalysatorbett und beim Austritt der Reaktionsmischung aus dem letzten Katalysatorbett.

In einer bevorzugten Ausführungsform erfolgt die Temperierung des Reaktorrohrs von außen mit einem Wärmeträgerstrom, wobei es sich bei dem Wärmeträger z. B. um ein Öl, eine Salzschmelze oder eine andere wärmeübertragende Flüssigkeit handeln kann.

Die erfindungsgemäße Reaktionsführung hat gegenüber einer Synthese in der flüssigen Phase und bevorzugt gegenüber einer nicht-isothermen Synthese in der Gasphase u.a. den Vorteil besserer Ausbeuten und einer höheren Sicherheit bezogen auf Durchgehreaktionen, insbesondere bei hohen Reaktionstemperaturen (z. B. 200 bis 300°C). Durch die, bevorzugt isotherme, Gasphasen-Fahrweise ist das Potential einer Durchgehreaktion während der Synthese stark reduziert. Die vorhandene Masse im Reaktor, die für eine Durchgehreaktion zur Verfügung stünde, ist nur ein Bruchteil der Masse eines Flüssigphasenverfahrens.

Das erfindungsgemäße Verfahren wird kontinuierlich durchgeführt, wobei der Katalysator bevorzugt als Festbett im Reaktor angeordnet ist. Dabei ist sowohl eine Anströmung des Katalysatorfestbetts von oben als auch von unten möglich. Der Gasstrom wird dabei durch Temperatur, Druck und Menge so eingestellt, dass auch schwerer siedende (hoch siedende) Reaktionsprodukte in der Gasphase verbleiben.

Die Aminkomponente (Stickstoffverbindung) wird bevorzugt in der 0,90- bis 100-fachen molaren Menge, insbesondere in der 1,0- bis 10-fachen molaren Menge, jeweils bezogen auf den/das eingesetzte/n Alkohol, Aldehyd und/oder Keton eingesetzt.

Das erfindungsgemäße Verfahren wird bevorzugt bei einem Absolutdruck im Bereich von 1 bis 300 bar, bevorzugt 1 bis 50 bar, besonders bevorzugt 1 bis 30 bar, durchgeführt.

Das erfindungsgemäße Verfahren wird im Fall einer Alkoholaminierung bevorzugt bei einer Temperatur im Bereich von 80 bis 300°C, bevorzugt 150 bis 250°C, besonders bevorzugt 170 bis 230°C, durchgeführt.

Das erfindungsgemäße Verfahren wird im Fall einer Aldehyd- und/oder Ketonaminierung bevorzugt bei einer Temperatur im Bereich von 60 bis 200°C, bevorzugt 80 bis 170°C, besonders bevorzugt 100 bis 150°C, durchgeführt.

Bevorzugt wird eine Abgasmenge von 5 bis 800 Normkubikmeter/h, insbesondere 20 bis 300 Normkubikmeter/h, gefahren.

[Normkubikmeter ($Nm^3$) = auf Normalbedingungen umgerechnetes Volumen].

Bevorzugt wird im erfindungsgemäßen Verfahren der Alkohol, Aldehyd und/oder das Keton als wässrige Lösung einsetzt.

Bevorzugt wird im erfindungsgemäßen Verfahren der Ammoniak, das primäre oder sekundäre Amin als wässrige Lösung einsetzt.

Die Katalysatorbelastung liegt bevorzugt im Bereich von 0,1 bis 2,0, bevorzugt 0,1 bis 1,0, besonders bevorzugt 0,2 bis 0,6, kg Alkohol, Aldehyd und/oder Keton pro Liter Katalysator (Schüttvolumen) und Stunde.

Die Anwendung höherer Temperaturen, höherer Gesamtdrücke und höherer Katalysatorbelastungen ist möglich. Der Druck im Reaktor, welcher sich aus der Summe der Partialdrücke des Aminierungsmittels, der Alkohol-, Aldehyd- und/oder Keton-Komponente und der gebildeten Reaktionsprodukte bei den angegebenen Temperaturen ergibt, wird zweckmäßigerweise durch Aufpressen von Wasserstoff auf den gewünschten Reaktionsdruck erhöht.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z. B. durch eine fraktionierende Rektifikation. Geeignete Aufarbeitungsverfahren sind z. B. in EP 1 312 600 A und EP 1 312 599 A (beide BASF AG) beschrieben. Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Als Aminierungsmittel im erfindungsgemäßen Verfahren neben Ammoniak geeignete primäre und sekundäre Amine sind Amine, die aufgrund ihrer Siedepunkte verfahrenstechnisch im Rahmen der Prozessparameter in der Gasphase gehalten werden können. Das Gleiche gilt für die Verfahrensproduktamine und die Verfahrensedukte (Alkohol, Aldehyd, Keton).

Mit dem erfindungsgemäßen Verfahren herstellbar sind z. B. Amine der Formel I

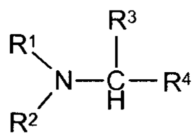

(I),

in der

$R^1$, $R^2$ — Wasserstoff (H), Alkyl, wie $C_{1-20}$-Alkyl, Cycloalkyl, wie $C_{3-12}$-Cycloalkyl, Alkoxyalkyl, wie $C_{2-30}$-Alkoxyalkyl, Dialkylaminoalkyl, wie $C_{3-30}$-Dialkylaminoalkyl, Aryl, Aralkyl, wie $C_{7-20}$-Aralkyl, und Alkylaryl, wie $C_{7-20}$-Alkylaryl, oder gemeinsam $-(CH_2)_j-X-(CH_2)_k-$,

$R^3$, $R^4$ — Wasserstoff (H), Alkyl, wie $C_{1-20}$-Alkyl, Cycloalkyl, wie $C_{3-12}$-Cycloalkyl, Hydroxyalkyl, wie $C_{1-20}$-Hydroxyalkyl, Aminoalkyl, wie $C_{1-20}$-Aminoalkyl, Hydroxyalkylaminoalkyl, wie $C_{2-20}$-Hydroxyalkylaminoalkyl, Alkoxyalkyl, wie $C_{2-30}$-Alkoxyalkyl, Dialkylaminoalkyl, wie $C_{3-30}$-Dialkylaminoalkyl, Alkylaminoalkyl, wie $C_{2-30}$-Alkylaminoalkyl, $R^5-(OCR^6R^7CR^8R^9)_n-(OCR^6R^7)$, Aryl, Heteroaryl, Aralkyl, wie $C_{7-20}$-Aralkyl, Heteroarylalkyl, wie $C_{4-20}$-Heteroarylalkyl, Alkylaryl, wie $C_{7-20}$-Alkylaryl, Alkylheteroaryl, wie $C_{4-20}$-Alkylheteroaryl, und $Y-(CH_2)_m-NR^5-(CH_2)_q$ oder gemeinsam $-(CH_2)_l-X-(CH_2)_m-$ oder

$R^2$ und $R^4$ — gemeinsam $-(CH_2)_l-X-(CH_2)_m-$,

$R^5$, $R^{10}$ — Wasserstoff (H), Alkyl, wie $C_{1-4}$-Alkyl, Alkylphenyl, wie $C_{7-40}$-Alkylphenyl,

| | |
|---|---|
| $R^6$, $R^7$, $R^8$, $R^9$ | Wasserstoff (H), Methyl oder Ethyl, |
| X | $CH_2$, $CHR^5$, Sauerstoff (O), Schwefel (S) oder $NR^5$, |
| Y | $N(R^{10})_2$, Hydroxy, $C_{2-20}$-Alkylaminoalkyl oder $C_{3-20}$-Dialkylaminoalkyl, |
| n | eine ganze Zahl von 1 bis 30 und |
| j, k, l, m, q | eine ganze Zahl von 1 bis 4, |

bedeuten.

Das erfindungsgemäße Verfahren findet daher bevorzugt zur Herstellung eines Amins I Anwendung, indem man einen primären oder sekundären Alkohol der Formel II

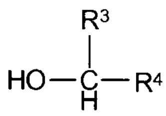

(II)

und/oder Aldehyd und/oder Keton der Formel VI bzw. VII

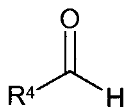

(VI)

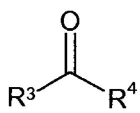

(VII)

mit einer Stickstoffverbindung der Formel III

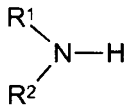

(III),

wobei $R^1$, $R^2$, $R^3$ und $R^4$ die oben genannten Bedeutungen haben, umsetzt.

Bei dem Eduktalkohol kann es sich auch um einen Aminoalkohol handeln, z. B. einem Aminoalkohol gemäß der Formel II.

Wie aus den Definitionen für die Reste $R^2$ und $R^4$ hervorgeht, kann die Umsetzung auch intramolekular in einem entsprechenden Aminoalkohol, Aminoketon oder Aminoaldehyd erfolgen.

Zur Herstellung des Amins I wird demnach rein formal ein Wasserstoffatom der Stickstoffverbindung III durch den Rest $R^4(R^3)CH$- unter Freisetzung von einem Moläquivalent Wasser ersetzt.

Das erfindungsgemäße Verfahren findet auch bevorzugt Anwendung bei der Herstellung eines zyklischen Amins der Formel IV

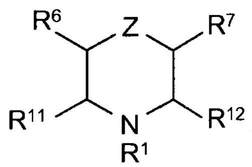

(IV),

in der

| | |
|---|---|
| $R^{11}$ und $R^{12}$ | Wasserstoff (H), Alkyl, wie $C_1$- bis $C_{20}$-Alkyl, Cycloalkyl, wie $C_3$- bis $C_{12}$-Cycloalkyl, Aryl, Heteroaryl, Aralkyl, wie $C_7$- bis $C_{20}$-Aralkyl, und Alkylaryl, wie $C_7$- bis $C_{20}$-Alkylaryl, |

Z CH$_2$, CHR$^5$, Sauerstoff (O), NR$^5$ oder NCH$_2$CH$_2$OH bedeuten und

R$^1$, R$^6$, R$^7$ die oben genannten Bedeutungen haben,
durch Umsetzung eines Alkohols der Formel V

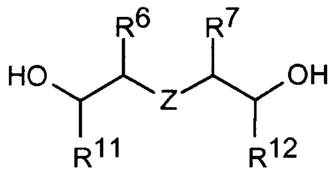

(V)

mit Ammoniak oder einem primären Amin der Formel VIII

$R^1$- $NH_2$ (VIII).

Die Substituenten R$^1$ bis R$^{12}$, die Variablen X, Y, Z und die Indizes j, k, l, m, n und q in den Verbindungen I, II, III, IV, V, VI, VII und VIII haben unabhängig voneinander folgende Bedeutungen:

R$^1$, R$^2$, R$^3$, R$^4$, R$^5$, R$^6$, R$^7$ R$^8$, R$^9$, R$^{10}$, R$^{11}$, R$^{12}$:

- Wasserstoff (H),

R$^3$, R$^4$:

- Alkyl, wie C$_{1-20}$-Alkyl, bevorzugt C$_{1-14}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl,

- Hydroxyalkyl, wie C$_{1-20}$-Hydroxyalkyl, bevorzugt C$_{1-8}$-Hydroxyalkyl, besonders bevorzugt C$_{1-4}$-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-(Hydroxymethyl)ethyl,

- Aminoalkyl, wie C$_{1-20}$-Aminoalkyl, bevorzugt C$_{1-8}$-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(2-Aminoethyl)-2-aminoethyl und N-(2-Aminoethyl)aminomethyl,

- Hydroxyalkylaminoalkyl, wie C$_{2-20}$-Hydroxyalkylaminoalkyl, bevorzugt C$_{3-8}$-Hydroxyalkylaminoalkyl, wie (2-Hydroxyethylamino)methyl, 2-(2-Hydroxyethylamino)ethyl und 3-(2-Hydroxyethylamino)propyl,

- $R^5$-$(OCR^6R^7CR^8R^9)_n$-$(OCR^6R^7)$, bevorzugt $R^5$-$(OCHR^7CHR^9)_n$-$(OCR^6R^7)$, besonders bevorzugt $R^5$-$(OCH_2CHR^9)_n$-$(OCR^6R^7)$,

- Alkylaminoalkyl, wie C$_{2-30}$-Alkylaminoalkyl, bevorzugt C$_{2-20}$-Alkylaminoalkyl, besonders bevorzugt C$_{2-8}$-Alkylaminoalkyl, wie Methylaminomethyl, 2-Methylaminoethyl, Ethylaminomethyl, 2-Ethylaminoethyl und 2-(iso-Propylamino)ethyl, $(R^5)HN$-$(CH_2)_q$,

- $Y$-$(CH_2)_m$-$NR^5$-$(CH_2)_q$,

- Heteroarylalkyl, wie C$_{4-20}$-Heteroarylalkyl, wie Pyrid-2-yl-methyl, Furan-2-ylmethyl, Pyrrol-3-yl-methyl und Imidazol-2-yl-methyl,

- Alkylheteroaryl, wie C$_{4-20}$-Alkylheteroaryl, wie 2-Methyl-3-pyridinyl, 4,5-Dimethyl-imidazol-2-yl, 3-Methyl-2-furanyl und 5-Methyl-2-pyrazinyl,

- Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,

$R^1$, $R^2$, R3, $R^4$:

- Cycloalkyl, wie $C_{3-12}$-Cycloalkyl, bevorzugt $C_{3-8}$-Cycloalkyl, wie Cyclopropyl, Cyclobutyl, Cyclopentyl, Cyclohexyl, Cycloheptyl und Cyclooctyl, besonders bevorzugt Cyclopentyl und Cyclohexyl,

- Alkoxyalkyl, wie $C_{2-30}$-Alkoxyalkyl, bevorzugt $C_{2-20}$-Alkoxyalkyl, besonders bevorzugt $C_{2-8}$-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxy-ethyl und 2-Methoxyethyl, besonders bevorzugt $C_{2-4}$-Alkoxyalkyl,

- Dialkylaminoalkyl, wie $C_{3-30}$-Dialkylaminoalkyl, bevorzugt $C_{3-20}$-Dialkylaminoalkyl, besonders bevorzugt $C_{3-10}$-Dialkylaminoalkyl, wie N,N-Dimethylaminomethyl, (N,N-Dibutylamino)methyl, 2-(N,N-Dimethylamino) ethyl, 2-(N,N-Diethylamino)ethyl, 2-(N,N-Dibutylamino)ethyl, 2-(N,N-Di-n-propylamino)ethyl und 2-(N,N-Di-iso-propylamino)ethyl, 3-(N,N-Dimethylamino)propyl, $(R^5)_2N-(CH_2)_q$,

- Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,

- Alkylaryl, wie $C_{7-20}$-Alkylaryl, bevorzugt $C_{7-12}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,

- Aralkyl, wie $C_{7-20}$-Aralkyl, bevorzugt $C_{7-12}$-Phenylalkyl, wie Benzyl, p-Methoxybenzyl, 3,4-Dimethoxybenzyl, 1-Phenethyl, 2-Phenethyl, 1-Phenylpropyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,

- $R^3$ und $R^4$ oder $R^2$ und $R^4$ gemeinsam eine $-(CH_2)_l-X-(CH_2)_m-$ Gruppe, wie-$(CH_2)_3$-, $-(CH_2)_4-$, $-(CH_2)_5-$, $-(CH_2)_6-$, $-(CH_2)_7-$, $-(CH_2)-O-(CH_2)_2-$, $-(CH_2)-NR^5-(CH_2)_2-$, $-(CH_2)-CHR^5-(CH_2)_2-$, $-(CH_2)_2-O-(CH_2)_2-$, $-(CH_2)_2-NR^5-(CH_2)_2-$, $-(CH_2)_2-CHR^5-(CH_2)_2-$, $-CH_2-O-(CH_2)_3-$, $-CH_2-NR^5-(CH_2)_3-$, $-CH_2-CHR^5-(CH_2)_3-$,

$R^1$, $R^2$:

- Alkyl, wie $C_{1-20}$-Alkyl, bevorzugt $C_{1-8}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, 2-Ethylhexyl, besonders bevorzugt $C_{1-4}$-Alkyl, oder

- $R^1$ und $R^2$ gemeinsam eine $-(CH_2)_j-X-(CH_2)_k-$ Gruppe, wie $-(CH_2)_3-$, $-(CH_2)_4-$,$-(CH_2)_5-$, $-(CH_2)_6-$, $-(CH_2)_7-$, $-(CH_2)-O-(CH_2)_2-$, $-(CH_2)-NR^5-(CH_2)_2-$, $-(CH_2)-CHR^5-(CH_2)_2-$, $-(CH_2)_2-O-(CH_2)_2-$, $-(CH_2)_2-NR^5-(CH_2)_2-$, $-(CH_2)_2-CHR^5-(CH_2)_2-$, $-CH_2-O-(CH_2)_3-$, $-CH_2-NR^5-(CH_2)_3-$, $-CH_2-CHR^5-(CH_2)_3-$,

$R^5$, $R^{10}$:

- Alkyl, bevorzugt $C_{1-4}$-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,

- Alkylphenyl, bevorzugt $C_{7-40}$-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl, insbesondere $C_{7-20}$-Alkylphenyl,

$R^6$, $R^7$, $R^8$, $R^9$:

- Methyl oder Ethyl, bevorzugt Methyl,

$R^{11}$, $R^{12}$.

- Alkyl, wie $C_1$- bis $C_{20}$-Alkyl, Cycloalkyl, wie $C_3$- bis $C_{12}$-Cycloalkyl, Aryl, Heteroaryl, Aralkyl, wie $C_7$- bis $C_{20}$-Aralkyl, und Alkylaryl, wie $C_7$- bis $C_{20}$-Alkylaryl, jeweils wie oben definiert,

X:

- $CH_2$, $CHR^5$, Sauerstoff (O), Schwefel (S) oder $NR^5$, bevorzugt $CH_2$ und O,

Y:

- $N(R^{10})_2$, bevorzugt $NH_2$ und $N(CH_3)_2$,
- Hydroxy (OH),
- $C_{2-20}$-Alkylaminoalkyl, bevorzugt $C_{2-16}$-Alkylaminoalkyl, wie Methylaminomethyl, 2-Methylaminoethyl, Ethylaminomethyl, 2-Ethylaminoethyl und 2-(iso-Propylamino)ethyl,
- $C_{3-20}$-Dialkylaminoalkyl, bevorzugt $C_{3-16}$-Dialkylaminoalkyl, wie Dimethylaminomethyl, 2-Dimethylaminoethyl, 2-Diethylaminoethyl, 2-(Di-n-propylamino)ethyl und 2-(Di-iso-propylamino)ethyl,

Z:

- $CH_2$, $CHR^5$, O, $NR^5$ oder $NCH_2CH_2OH$,

j, l:

- eine ganze Zahl von 1 bis 4 (1, 2, 3 oder 4), bevorzugt 2 und 3, besonders bevorzugt 2,

k, m, q:

- eine ganze Zahl von 1 bis 4 (1, 2, 3 oder 4), bevorzugt 2, 3 und 4, besonders bevorzugt 2 und 3,

n:

- eine ganze Zahl von 1 bis 30, bevorzugt eine ganze Zahl von 1 bis 8 (1, 2, 3, 4, 5, 6, 7 oder 8), besonders bevorzugt eine ganze Zahl von 1 bis 6.

Als Alkohole eignen sich unter den o.g. Voraussetzungen praktisch alle primären und sekundären Alkohole mit aliphatischer OH-Funktion. Die Alkohole können geradkettig, verzweigt oder zyklisch sein. Sekundäre Alkohole werden ebenso aminiert wie primäre Alkohole. Die Alkohole können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Alkohole aminiert werden, so hat man es über die Steuerung der Reaktionsbedingungen in der Hand, bevorzugt Aminoalkohole, zyklische Amine oder mehrfach aminierte Produkte zu erhalten.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom (Pyrrolidinen).

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom (Hexamethyleniminen).

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom (Piperidinen). Aus Diglykol kann demnach durch Aminierung mit $NH_3$ Monoaminodiglykol (= ADG = $H_2N-CH_2CH_2-O-CH_2CH_2-OH$), Diaminodiglykol oder besonders bevorzugt Morpholin erhalten werden. Aus Diethanolamin wird entsprechend besonders bevorzugt Piperazin erhalten. Aus Triethanolamin kann N-(2-Hydroxyethyl)-piperazin erhalten werden.

Bevorzugt werden beispielsweise die folgenden Alkohole aminiert:

Methanol, Ethanol, n-Propanol, Isopropanol, n-Butanol, iso-Butanol, n-Pentanol, n-Hexanol, 2-Ethylhexanol, Tridecanol, Stearylalkohol, Palmitylalkohol, Cyclobutanol, Cyclopentanol, Cyclohexanol, Benzylalkohol, 2-Phenyl-ethanol, 2-(p-Methoxyphenyl)-ethanol, 2-(3,4-Dimethoxyphenyl)ethanol, 1-Phenyl-3-butanol, Ethanolamin, n-Propanolamin, Isopropanolamin, 2-Amino-1-propanol, 1-Methoxy-2-propanol, 3-Amino-2,2-dimethyl-1-propanol, n-Pentanolamin (1-Amino-5-pentanol), n-Hexanolamin (1-Amino-6-hexanol), Ethanolamin, Diethanolamin, Triethanolamin, N-Alkyldiethanolamine, Diisopropanolamin, 3-(2-Hydroxyethylamino)propan-1-ol, 2-(N,N-Dimethylamino)ethanol,

2-(N,N-Diethylamino)ethanol, 2-(N,N-Di-n-propylamino)ethanol, 2-(N,N-Di-isopropylamino)ethanol, 2-(N,N-Di-n-butylamino)ethanol, 2-(N,N-Di-iso-butylamino)-ethanol, 2-(N,N-Di-sec.-butylamino)ethanol, 2-(N,N-Di-tert.-butylamino)ethanol, 3-(N,N-Dimethylamino)propanol, 3-(N,N-Diethylamino)propanol, 3-(N,N-Di-n-propylamino)-propanol, 3-(N,N-Di-iso-propylamino)propanol, 3-(N,N-Di-n-butylamino)propanol, 3-(N,N-Di-iso-butylamino)propanol, 3-(N,N-Di-sec.-butylamino)propanol, 3-(N,N-Di-tert.-butylamino)propanol, 1-Dimethylamino-pentanol-4, 1-Diethylamino-pentanol-4, Ethylenglykol, 1,2-Propylenglykol, 1,3-Propylenglykol, Diglykol, 1,4-Butandiol, 1,5-Pentandiol, 1,6-Hexandiol, 2,2-Bis[4-hydroxycyclohexyl]propan, Methoxyethanol, Propoxyethanol, Butoxyethanol, Polyisobutylalkohole, Polypropylalkohole, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Besonders bevorzugte Alkohole sind Methanol, Ethanol, n-Propanol, i-Propanol, n-Butanol, sek.-Butanol, 1,4-Butandiol, 1,5-Pentandiol, 1,6-Hexandiol, 2-Ethylhexanol, Cyclohexanol, Fettalkohole, Ethylenglykol, Diethylenglykol (DEG), Triethylenglykol (TEG), 2-(2-Dimethylamino-ethoxy)ethanol, N-Methyldiethanolamin und 2-(2-Dimethylaminoethoxy)ethanol.

Als im erfindungsgemäßen Verfahren einsetzbare Ketone eignen sich unter den o.g. Voraussetzungen praktisch alle aliphatischen und aromatischen Ketone. Die aliphatischen Ketone können geradkettig, verzweigt oder zyklisch sein, die Ketone können Heteroatome enthalten. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden beispielsweise die folgenden Ketone aminierend hydriert:

Aceton, Ethylmethylketon, Methylvinylketon, Isobutylmethylketon, Butanon, 3-Methylbutan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich unter den o.g. Voraussetzungen praktisch alle aliphatischen und aromatischen Aldehyde. Die aliphatischen Aldehyde können geradkettig, verzweigt oder zyklisch sein, die Aldehyde können Heteroatome enthalten. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)-acetaldehyd, 4-Formyltetrahydropyran, 3- Formyltetrahydrofuran, 5-Formylvaleronitril, Citronellal, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Alkoholen, Aldehyden oder Ketonen in Gegenwart von Wasserstoff können sowohl Ammoniak als auch primäre oder sekundäre, aliphatische oder cycloaliphatische oder aromatische Amine eingesetzt werden.

Bei Verwendung von Ammoniak als Aminierungsmittel wird die alkoholische Hydroxylgruppe bzw. die Aldehydgruppe bzw. die Ketogruppe zunächst in die primäre Aminogruppen ($-NH_2$) umgewandelt. Das so gebildete primäre Amin kann mit weiterem Alkohol bzw. Aldehyd bzw. Keton zu dem entsprechenden sekundären Amin und diese wiederum mit weiterem Alkohol bzw. Aldehyd bzw. Keton zu dem entsprechenden, vorzugsweise symmetrischen, tertiären Amin reagieren. Je nach Zusammensetzung des Reaktionsansatzes oder des Eduktstroms (bei kontinuierlicher Fahrweise)

und je nach den angewandten Reaktionsbedingungen - Druck, Temperatur, Reaktionszeit (Katalysatorbelastung) - lassen sich auf diese Weise je nach Wunsch bevorzugt primäre, sekundäre oder tertiäre Amine darstellen.

Aus mehrwertigen Alkoholen bzw. Di- oder Oligoaldehyden bzw. Di- oder Oligoketonen bzw. Ketoaldehyden lassen sich auf diese Weise durch intramolekulare hydrierende Aminierung zyklische Amine wie z. B. Pyrrolidine, Piperidine, Hexamethylenimine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie Ethyldiisopropylamin und Ethyldicyclohexylamin verwendet. Beispielsweise werden die folgenden Mono- und Dialkylamine als Aminierungsmittel verwendet: Methylamin, Dimethylamin, Ethylamin, Diethylamin, n-Propyl-amin, Di-n-propyl-amin, iso-Propylamin, Di-isopropyl-amin, Isopropylethylamin, n-Butylamin, Di-n-Butylamin, s-Butylamin, Di-s-Butylamin, iso-Butylamin, n-Pentylamin, s-Pentylamin, iso-Pentylamin, n-Hexylamin, s-Hexylamin, iso-Hexylamin, Cyclohexylamin, Anilin, Toluidin, Piperidin, Morpholin und Pyrrolidin.

Mit dem erfindungsgemäßen Verfahren besonders bevorzugt hergestellte Amine sind zum Beispiel Morpholin (aus Aminodiglykol), Morpholin und/oder 2,2'-Dimorpholinodiethylether (DMDEE) (aus DEG und Ammoniak), 6-Dimethylaminohexanol-1 (aus Hexandiol und Dimethylamin (DMA)), Triethylamin (aus Ethanol und Diethylamin (DEA)), Dimethylethylamin (aus Ethanol und DMA), N-($C_{1-4}$-alkyl)morpholin (aus DEG und Mono($C_{1-4}$-alkyl)amin), N-($C_{1-4}$-alkyl) piperidin (aus 1,5-Pentandiol und Mono($C_{1-4}$-alkyl)amin), Piperazin (aus Aminoethylethanolamin (AEEA) und Ammoniak), N-Methylpiperazin (aus Diethanolamin und MMA), N,N'-Dimethylpiperazin (aus N-Methyldiethanolamin und MMA), Ethylendiamin (EDA) und/oder Diethylentriamin (DETA) und/oder PIP (aus Monoethanolamin (MEOA) und Ammoniak), 2-Ethylhexylamin und Bis(2-Ethylhexyl)amin (aus 2-Ethylhexanol und $NH_3$), Tridecylamin und Bis(Tridecyl)-amin (aus Tridecanol und $NH_3$), n-Octylamin (aus n-Octanol und $NH_3$), 1,2-Propylendiamin (aus 2-Hydroxy-propylamin und $NH_3$), 1-Diethylamino-4-aminopentan (aus 1-Diethylamino-4-hydroxypentan und $NH_3$), N,N-Di($C_1$-$_4$-alkyl)cyclohexylamin (aus Cyclohexanon und/oder Cyclohexanol und Di($C_{1-4}$-alkyl)amin), Polyisobutenamin (aus PibOxo und $NH_3$), n-Propylamine (wie Mono-/Dipropylamin, Dimethylpropylamin) (aus Propionaldehyd und/oder n-Propanol und $NH_3$ bzw. DMA), N,N-Dimethyl-N-isopropylamin (aus i-Propanol und/oder Aceton und DMA), N,N-Dimethyl-N-butylamine (1-, 2-oder iso-Butanol und/oder Butanal, i-Butanal oder Butanon und DMA), 2-(2-Di($C_{1-4}$-alkyl)aminoethoxy)ethanol und/oder Bis(2-di ($C_{1-4}$-alkyl)aminoethyl)ether (aus DEG und Di($C_{1-4}$-alkyl)amin), 1,2-Ethylendiamin (EDA), Diethylentriamin (DETA) und/ oder Piperazin (PIP) (aus Monoethylenglykol (MEG) und Ammoniak), 1,8-Diamino-3,6-dioxaoctan und/oder 1-Amino-8-hydroxy-3,6-dioxa-octan (aus Triethylenglykol (TEG) und Ammoniak), 1-Methoxy-2-propylamin (1-Methoxy-isopropylamin, MOIPA) (aus 1-Methoxy-2-propanol und Ammoniak).

## Beispiele

Für die folgenden Beispiele wurde ein Kupfer-Katalysator der Zusammensetzung 55 Gew.-% CuO und 45 Gew.-% gamma-$Al_2O_3$ (nach dessen letzter Wärmebehandlung und vor dessen Reduktion mit Wasserstoff) verwendet.

Die Herstellung des Katalysators erfolgte durch Tränkung von gamma-$Al_2O_3$ - Pulver mit einer wässrigen Kupfernitrat-Lösung. Die Tablettierung erfolgte nach üblicher Methode. Vor Beginn der Umsetzung wurde der Katalysator im Wasserstoffstrom bei ca. 200°C reduziert (s.u.).

Die Versuche wurden in von unten nach oben durchströmten Gasphasenöfen-Reaktoren in kontinuierlicher Fahrweise entweder in einem 3,5 m langen ölbeheizten Doppelmantelrohr mit 4 cm Innendurchmesser durchgeführt, welches von unten nach oben mit 180 ml Keramikkugeln (6-9 mm), 1 Liter Katalysator und 3,3 Liter Inertmaterial (V2A-Ringe, 6 mm Durchmesser) befüllt wurde, oder in einem 2,1 m langen ölbeheizten Doppelmantelrohr mit 4,11 cm Innendurchmesser, welches von unten nach oben mit 20 ml Keramikkugeln (6-9 mm), 1 Liter Katalysator und 1,33 Liter Inertmaterial (V2A-Ringe, 6 mm Durchmesser) befüllt wurde. Die Reaktoren wurden bei 20 bzw. 25 bar betrieben.

Die Katalysator-Formkörper in Tablettenform wurden in den Größen 5 x 5 mm (5 mal 5 mm, d.h. 5 mm Durchmesser und 5 mm Höhe), 3 x 3 mm sowie 1,5 x 1,5 mm verwendet. Nach dem Einbau in den Reaktor wurden alle Katalysatoren nach folgender Vorschrift drucklos aktiviert:

12 h bei 180 °C (Ölkreis Reaktor) mit 20 Nl/h $H_2$ und 400 Nl/h $N_2$, 12 h bei 200 °C mit 20 Nl/h $H_2$ und 400 Nl/h $N_2$, über 6 h $N_2$ durch 200 Nl/h $H_2$ ersetzen, 6 h bei 200 °C mit 200 Nl/h $H_2$.
(Nl = Normliter = auf Normalbedingungen umgerechnetes Volumen).

Die Zuläufe Frischwasserstoff, Kreisgas, Druckgase und Edukte wurden über ein System von drei Rohrschlangenwärmetauschern auf die gewünschte Reaktortemperatur erhitzt. Der dritte Wärmetauscher wurde über einen Temperaturfühler kurz vor dem Reaktor geregelt. Die Ölbeheizung des Doppelmantelreaktors wurde ebenfalls auf die gewünschte Reaktortemperatur eingestellt. Über zwei weitere Rohrschlangenwärmetauscher wurde der Reaktoraustrag zunächst mit Flusswasser, anschließend mit einem Kryostaten auf 10 °C abgekühlt und in einen Druckabscheider gefahren. Dort erfolgte die Trennung von Flüssig- und Gasphase. Die Flüssigphase wurde in einen auf 30 °C temperierten

Niederdruckabscheider entspannt, von wo aus die Sprudelgase über das Abgas abgeführt und die Flüssigkeit in das Austragsfass gefördert wurden. Über einen Kreisgasverdichter wurde die Gasphase aus dem Druckabscheider in definierter Menge zurückgeführt und diente erneut als Trägergas für die Einsatzstoffe. Eine Druckregelung sorgte dafür, dass überschüssiges Gas auf die Muffel zur Verbrennung gefahren wurde. Umsatz und Selektivität des Austrages wurden durch gaschromatographische Analyse bestimmt.

Beispiel 1

Die verschiedenen Formkörper wurden in der Umsetzung von Diethylenglykol (DEG) mit Dimethylamin (DMA) zu Bis-(dimethylaminoethyl)-ether (BDMAE) bei 210 °C, einem Anlagendruck von 20 bar, einer DEG-Belastung von 0,5 kg/Liter•h, einer Kreisgasmenge von 10 $Nm^3/h$ und 300 Nl/h Frischwasserstoff untersucht.
($Nm^3$ = Normkubikmeter = auf Normalbedingungen umgerechnetes Volumen).
Es wurde beobachtet, dass sich mit der schrittweisen Verkleinerung der Formkörper von 5 x 5 mm über 3 x 3 mm auf 1,5 x 1,5 mm sowohl Umsatz als auch Selektivität der Aminierungsreaktion verbesserten. Der Umsatz erhöhte sich dabei um bis zu 5 %, die Selektivität um bis zu 5 % beim Vergleich der 5 x 5 mm zu den 1,5 x 1,5 mm Formkörpern. Bei Verwendung der 1,5 x 1,5 mm Formkörper konnte der Gehalt an BDMAE im Syntheseaustrag durch Erhöhung der eingesetzten DMA Menge von 29 Gew.-% (5 x 5 mm) auf über 40 Gew.-% (1,5 x 1,5 mm) verbessert werden, ohne dass eine signifikante Steigerung störender Nebenkomponenten zu beobachten war. Das heißt, die Tendenz zum Scrambling von DMA zu Monomethylamin (MMA) und Trimethylamin (TMA), die bei entsprechender Erhöhung des DMA-Anteils mit den 5 x 5 cm Formkörpern auftritt, war vermindert.

Beispiel 2

Formkörper der Größe 3 x 3 mm und 5 x 5 mm wurden in der Umsetzung von Butandiol mit Ammoniak zu Pyrrolidin bei 240 °C, einem Anlagendruck von 20 bar und einer Butandiol Belastung von 0,29 kg/Liter•h untersucht. Es wurde eine Selektivitätssteigerung zum Reaktionsprodukt um 8 % auf 89 % bei Verwendung der 3 x 3 mm Formkörper im Vergleich zu den 5 x 5 mm Formkörpern beobachtet.

Beispiel 3

Formkörper der Größe 3 x 3 mm und 5 x 5 mm wurden in der Umsetzung von Pentandiol mit Ammoniak zu Piperidin bei 220-240 °C, einem Anlagendruck von 20 bar und einer Pentandiol Belastung von 0,31 kg/Liter•h untersucht. Es wurde eine Selektivitätssteigerung zum Reaktionsprodukt um mehr als 10 % auf ca. 92 % bei Verwendung der 3 x 3 mm Formkörper im Vergleich zu den 5 x 5 mm Formkörpern beobachtet.

Beispiel 4

Formkörper der Größe 3 x 3 mm und 5 x 5 mm wurden in der Umsetzung von Propanal mit Dimethylamin zu N,N-Dimethylpropylamin bei 100 °C, einem Anlagendruck von 20 bar und einer Belastung von 0,32 kg/Liter•h untersucht. Es wurde eine Selektivitätssteigerung zum Reaktionsprodukt um 2 % auf 95 % bei Verwendung der 3x3 mm Formkörper im Vergleich zu den 5 x 5 mm Formkörpern beobachtet.

## Patentansprüche

1. Verfahren zur kontinuierlichen Herstellung eines Amins durch Umsetzung eines primären oder sekundären Alkohols mit aliphatischer OH-Funktion, Aldehyds und/oder Ketons mit Wasserstoff und einer Stickstoffverbindung, ausgewählt aus der Gruppe Ammoniak, primäre und sekundäre Amine, bei einer Temperatur im Bereich von 60 bis 300°C in Gegenwart eines kupfer- und aluminiumoxidhaltigen Katalysators, **dadurch gekennzeichnet, dass** die Umsetzung in der Gasphase erfolgt und die katalytisch aktive Masse des Katalysators vor dessen Reduktion mit Wasserstoff

    20 bis 75 Gew.% Aluminiumoxid,
    20 bis 75 Gew.-% sauerstoffhaltige Verbindungen des Kupfers, berechnet als CuO,
    0 bis 2 Gew.% sauerstoffhaltige Verbindungen des Natriums, berechnet als $Na_2O$, und

    weniger als 1 Gew.-% sauerstoffhaltige Verbindungen des Nickels, berechnet als NiO, enthält
    und der Katalysatorformkörper eine Tablettenform, mit einem Durchmesser im Bereich von 1,1 bis 3,5 mm und einer Höhe im Bereich von 1,1 bis 3,5 mm, aufweist.

2. Verfahren nach Anspruch 1, **dadurch gekennzeichnet, dass** der Katalysatorformkörper eine Tablettenform, mit einem Durchmesser im Bereich von 1,2 bis 3,2 mm und einer Höhe im Bereich von 1,2 bis 3,2 mm, aufweist.

3. Verfahren nach einem der vorhergehenden Ansprüche, **dadurch gekennzeichnet, dass** die katalytisch aktive Masse des Katalysators vor dessen Reduktion mit Wasserstoff weniger als 1 Gew.-% sauerstoffhaltige Verbindungen des Kobalts, berechnet als CoO, enthält.

4. Verfahren nach einem der vorhergehenden Ansprüche, **dadurch gekennzeichnet, dass** die katalytisch aktive Masse des Katalysators vor dessen Reduktion mit Wasserstoff

   25 bis 65 Gew.% Aluminiumoxid und
   30 bis 70 Gew.% sauerstoffhaltige Verbindungen des Kupfers, berechnet als CuO, enthält.

5. Verfahren nach einem der vorhergehenden Ansprüche, **dadurch gekennzeichnet, dass** die katalytisch aktive Masse des Katalysators vor dessen Reduktion mit Wasserstoff 0,05 bis 1 Gew.% sauerstoffhaltige Verbindungen des Natriums, berechnet als $Na_2O$, enthält.

6. Verfahren nach einem der vorhergehenden Ansprüche, **dadurch gekennzeichnet, dass** die katalytisch aktive Masse des Katalysators kein Nickel, Kobalt und/oder Ruthenium enthält.

7. Verfahren nach einem der vorhergehenden Ansprüche, **dadurch gekennzeichnet, dass** die Umsetzung isotherm, mit einer Temperaturabweichung von maximal +/-8°C erfolgt.

8. Verfahren nach einem der vorhergehenden Ansprüche, **dadurch gekennzeichnet, dass** die Umsetzung in einem Rohrreaktor erfolgt.

9. Verfahren nach dem vorhergehenden Anspruch, **dadurch gekennzeichnet, dass** die Umsetzung im Rohrreaktor bei einer Kreisgasfahrweise erfolgt.

10. Verfahren nach dem vorhergehenden Anspruch, **dadurch gekennzeichnet, dass** die Kreisgasmenge im Bereich von 40 bis 1500 $m^3$ (bei Betriebsdruck) / [$m^3$ Katalysator (Schüttvolumen) • h] liegt.

11. Verfahren nach einem der vorhergehenden Ansprüche, **dadurch gekennzeichnet, dass** die Umsetzung in einem Rohrbündelreaktor oder in einer Monostranganlage erfolgt.

12. Verfahren nach einem der vorhergehenden Ansprüche, **dadurch gekennzeichnet, dass** man die Umsetzung bei einem Absolutdruck im Bereich von 1 bis 300 bar durchführt.

13. Verfahren nach einem der Ansprüche 8 bis 12, **dadurch gekennzeichnet, dass** die Temperierung des Reaktorrohrs/der Reaktorrohre von außen mit einem Ölstrom oder einer Salzschmelze erfolgt.

14. Verfahren nach einem der Ansprüche 9 bis 13, **dadurch gekennzeichnet, dass** das Kreisgas mindestens 10 Vol. % Wasserstoff ($H_2$) enthält.

15. Verfahren nach einem der vorhergehenden Ansprüche, **dadurch gekennzeichnet, dass** die Stickstoffverbindung in der 0,90- bis 100-fachen molaren Menge bezogen auf den/das eingesetzte/n Alkohol, Aldehyd und/oder Keton eingesetzt wird.

16. Verfahren nach einem der vorhergehenden Ansprüche, **dadurch gekennzeichnet, dass** der Katalysator im Reaktor als Festbett angeordnet ist.

17. Verfahren nach einem der vorhergehenden Ansprüche, **dadurch gekennzeichnet, dass** man den Alkohol, Aldehyd und/oder das Keton als wässrige Lösung einsetzt.

18. Verfahren nach einem der vorhergehenden Ansprüche, **dadurch gekennzeichnet, dass** man den Ammoniak, das primäre oder sekundäre Amin als wässrige Lösung einsetzt.

19. Verfahren nach einem der vorhergehenden Ansprüche zur Herstellung von Morpholin und/oder 2,2'-Dimorpholinodiethylether durch Umsetzung von Diethylenglykol (DEG) mit Ammoniak.

20. Verfahren nach einem der Ansprüche 1 bis 18 zur Herstellung von N,N-Di($C_{1-4}$-alkyl)cyclohexylamin durch Umsetzung von Cyclohexanon und/oder Cyclohexanol mit Di($C_{1-4}$-alkyl)amin.

21. Verfahren nach einem der Ansprüche 1 bis 18 zur Herstellung von Piperidin durch Umsetzung von 1,5-Pentandiol mit Ammoniak.

22. Verfahren nach einem der Ansprüche 1 bis 18 zur Herstellung von Pyrrolidin durch Umsetzung von 1,4-Butandiol mit Ammoniak.

23. Verfahren nach einem der Ansprüche 1 bis 18 zur Herstellung von Hexamethylenimin durch Umsetzung von 1,6-Hexandiol mit Ammoniak.

24. Verfahren nach einem der Ansprüche 1 bis 18 zur Herstellung von N-($C_{1-4}$- alkyl)-morpholin durch Umsetzung von Diethylenglykol mit Mono($C_{1-4}$-alkyl)amin.

25. Verfahren nach dem vorhergehenden Anspruch zur Herstellung von N-Ethylmorpholin.

26. Verfahren nach einem der Ansprüche 1 bis 18 zur Herstellung von N-($C_{1-4}$-alkyl)-piperidin durch Umsetzung von 1,5-Pentandiol mit Mono($C_{1-4}$-alkyl)amin.

27. Verfahren nach einem der Ansprüche 1 bis 18 zur Herstellung von N,N-Dimethylethylamin durch Umsetzung von Ethanol mit Dimethylamin.

28. Verfahren nach einem der Ansprüche 1 bis 18 zur Herstellung von N,N-Dimethyl-N-propylamin durch Umsetzung von n-Propanol und/oder Propanal mit Dimethylamin (DMA).

29. Verfahren nach einem der Ansprüche 1 bis 18 zur Herstellung von N,N-Dimethyl-N-isopropylamin durch Umsetzung von i-Propanol und/oder Aceton mit DMA.

30. Verfahren nach einem der Ansprüche 1 bis 18 zur Herstellung von N,N-Dimethyl-N-(n-butyl)amin durch Umsetzung von 1-Butanol und/oder Butanal mit DMA.

31. Verfahren nach einem der Ansprüche 1 bis 18 zur Herstellung von N,N-Dimethyl-N-(iso-butyl)amin durch Umsetzung von i-Butanol und/oder i-Butanal mit DMA.

32. Verfahren nach einem der Ansprüche 1 bis 18 zur Herstellung von N,N-Dimethyl-N-(2-butyl)amin durch Umsetzung von 2-Butanol und/oder Butanon mit DMA.

33. Verfahren nach einem der Ansprüche 1 bis 18 zur Herstellung von Ethylpropylamin durch Umsetzung von n-Propanol und/oder Propanal mit Monoethylamin.

34. Verfahren nach einem der Ansprüche 1 bis 18 zur Herstellung von 2-(2-Di($C_{1-4}$-alkyl)aminoethoxy)ethanol und/oder Bis(2-di($C_{1-4}$-alkyl)aminoethyl)ether durch Umsetzung von Diethylenglykol mit Di($C_{1-4}$-alkyl)amin.

35. Verfahren nach einem der Ansprüche 1 bis 18 zur Herstellung von Piperazin durch Umsetzung von Aminoethylethanolamin (AEEA) mit Ammoniak.

36. Verfahren nach einem der Ansprüche 1 bis 18 zur Herstellung von 1,2-Ethylendiamin (EDA), Diethylentriamin (DETA) und/oder Piperazin (PIP) durch Umsetzung von Monoethylenglykol (MEG) mit Ammoniak.

37. Verfahren nach einem der Ansprüche 1 bis 18 zur Herstellung von 1,8-Diamino-3,6-dioxa-octan und/oder 1-Amino-8-hydroxy-3,6-dioxa-octan durch Umsetzung von Triethylenglykol (TEG) mit Ammoniak.

38. Verfahren nach einem der Ansprüche 1 bis 18 zur Herstellung von 1-Methoxy-2-propylamin (1-Methoxy-isopropylamin, MOIPA) durch Umsetzung von 1-Methoxy-2-propanol mit Ammoniak.

## Claims

1. A process for the continuous preparation of an amine by reaction of a primary or secondary alcohol having an aliphatic OH function, aldehyde and/or ketone with hydrogen and a nitrogen compound selected from the group consisting of ammonia, primary and secondary amines at a temperature in the range from 60 to 300°C in the presence of a catalyst comprising copper oxide and aluminum oxide, wherein the reaction takes place in the gas phase and the catalytically active composition of the catalyst before reduction with hydrogen comprises
from 20 to 75% by weight of aluminum oxide,
from 20 to 75% by weight of oxygen-comprising compounds of copper, calculated as CuO,
from 0 to 2% by weight of oxygen-comprising compounds of sodium, calculated as $Na_2O$, and
less than 1% by weight of oxygen-comprising compounds of nickel, calculated as NiO,
and the shaped catalyst body has a pellet shape having a diameter in the range from 1.1 to 3.5 mm and a height in the range from 1.1 to 3.5 mm.

2. The process according to claim 1, wherein the shaped catalyst body has a pellet shape having a diameter in the range from 1.2 to 3.2 mm and a height in the range from 1.2 to 3.2 mm.

3. The process according to either of the preceding claims, wherein the catalytically active composition of the catalyst before reduction with hydrogen comprises less than 1% by weight of oxygen-comprising compounds of cobalt, calculated as CoO.

4. The process according to any of the preceding claims, wherein the catalytically active composition of the catalyst before reduction with hydrogen comprises
from 25 to 65% by weight of aluminum oxide and from 30 to 70% by weight of oxygen-comprising compounds of copper, calculated as CuO.

5. The process according to any of the preceding claims, wherein the catalytically active composition of the catalyst before reduction with hydrogen comprises from 0.05 to 1% by weight of oxygen-comprising compounds of sodium, calculated as $Na_2O$.

6. The process according to any of the preceding claims, wherein the catalytically active composition of the catalyst comprises no nickel, cobalt and/or ruthenium.

7. The process according to any of the preceding claims, wherein the reaction is carried out isothermally with a temperature deviation of not more than +/- 8°C.

8. The process according to any of the preceding claims, wherein the reaction is carried out in a tube reactor.

9. The process according to the preceding claim, wherein the reaction is carried out in the tube reactor in a gas recycle mode.

10. The process according to the preceding claim, wherein the circulating gas flow is in the range from 40 to 1500 $m^3$ (at operating pressure)/[$m^3$ of catalyst (bed volume) ● h].

11. The process according to any of the preceding claims, wherein the reaction is carried out in a shell-and-tube reactor or in a monostream plant.

12. The process according to any of the preceding claims, wherein the reaction is carried out at an absolute pressure in the range from 1 to 300 bar.

13. The process according to any of claims 8 to 12, wherein the temperature of the reactor tube or tubes is maintained from the outside by means of an oil stream or a salt melt.

14. The process according to any of claims 9 to 13, wherein the circulating gas comprises at least 10% by volume of hydrogen ($H_2$).

15. The process according to any of the preceding claims, wherein the nitrogen compound is used in a from 0.90- to 100-fold molar amount based on the alcohol, aldehyde and/or ketone used.

**16.** The process according to any of the preceding claims, wherein the catalyst is arranged as a fixed bed in the reactor.

**17.** The process according to any of the preceding claims, wherein the alcohol, aldehyde and/or ketone are/is used as aqueous solution.

**18.** The process according to any of the preceding claims, wherein the ammonia or the primary or secondary amine is used as aqueous solution.

**19.** The process according to any of the preceding claims for the preparation of morpholine and/or bis(2-morpholinoethyl) ether by reaction of diethylene glycol (DEG) with ammonia.

**20.** The process according to any of claims 1 to 18 for the preparation of N,N-di($C_{1-4}$-alkyl)cyclohexylamine by reaction of cyclohexanone and/or cyclohexanol with di ($C_{1-4}$-alkyl) amine.

**21.** The process according to any of claims 1 to 18 for the preparation of piperidine by reaction of 1,5-pentanediol with ammonia.

**22.** The process according to any of claims 1 to 18 for the preparation of pyrrolidine by reaction of 1,4-butanediol with ammonia.

**23.** The process according to any of claims 1 to 18 for the preparation of hexamethylenimine by reaction of 1,6-hexanediol with ammonia.

**24.** The process according to any of claims 1 to 18 for the preparation of N-($C_{1-4}$-alkyl)morpholine by reaction of diethylene glycol with mono ($C_{1-4}$-alkyl) amine.

**25.** The process according to the preceding claim for the preparation of N-ethylmorpholine.

**26.** The process according to any of claims 1 to 18 for the preparation of N-($C_{1-4}$-alkyl)piperidine by reaction of 1,5-pentanediol with mono($C_{1-4}$-alkyl)amine.

**27.** The process according to any of claims 1 to 18 for the preparation of N,N-dimethylethylamine by reaction of ethanol with dimethylamine.

**28.** The process according to any of claims 1 to 18 for the preparation of N,N-dimethyl-N-propylamine by reaction of n-propanol and/or propanal with dimethylamine (DMA).

**29.** The process according to any of claims 1 to 18 for the preparation of N,N-dimethyl-N-isopropylamine by reaction of i-propanol and/or acetone with DMA.

**30.** The process according to any of claims 1 to 18 for the preparation of N,N-dimethyl-N-(n-butyl)amine by reaction of 1-butanol and/or butanal with DMA.

**31.** The process according to any of claims 1 to 18 for the preparation of N,N-dimethyl-N-(isobutyl)amine by reaction of i-butanol and/or i-butanal with DMA.

**32.** The process according to any of claims 1 to 18 for the preparation of N,N-dimethyl-N-(2-butyl)amine by reaction of 2-butanol and/or butanone with DMA.

**33.** The process according to any of claims 1 to 18 for the preparation of ethylpropylamine by reaction of n-propanol and/or propanal with monoethylamine.

**34.** The process according to any of claims 1 to 18 for the preparation of 2-(2-di($C_{1-4}$-alkyl)aminoethoxy)ethanol and/or bis(2-di($C_{1-4}$-alkyl)aminoethyl) ether by reaction of diethylene glycol with di($C_{1-4}$-alkyl)amine.

**35.** The process according to any of claims 1 to 18 for the preparation of piperazine by reaction of aminoethylethanolamine (AEEA) with ammonia.

**36.** The process according to any of claims 1 to 18 for the preparation of 1,2-ethylenediamine (EDA), diethylenetriamine (DETA) and/or piperazine (PIP) by reaction of monoethylene glycol (MEG) with ammonia.

**37.** The process according to any of claims 1 to 18 for the preparation of 1,8-diamino-3,6-dioxaoctane and/or 1-amino-8-hydroxy-3,6-dioxaoctane by reaction of triethylene glycol (TEG) with ammonia.

**38.** The process according to any of claims 1 to 18 for the preparation of 1-methoxy-2-propylamine (1-methoxyisopropylamine, MOIPA) by reaction of 1-methoxy-2-propanol with ammonia.

**Revendications**

**1.** Procédé de fabrication continue d'une amine par mise en réaction d'un alcool primaire ou secondaire contenant une fonction OH aliphatique, d'un aldéhyde et/ou d'une cétone avec de l'hydrogène et un composé azoté choisi dans le groupe constitué par l'ammoniac, les amines primaires et secondaires, à une température dans la plage allant de 60 à 300 °C en présence d'un catalyseur contenant de l'oxyde de cuivre et d'aluminium, **caractérisé en ce que** la réaction a lieu en phase gazeuse et la masse catalytiquement active du catalyseur contient, avant sa réduction avec de l'hydrogène,
20 à 75 % en poids d'oxyde d'aluminium,
20 à 75 % en poids de composés oxygénés de cuivre, calculés en tant que CuO,
0 à 2 % en poids de composés oxygénés de sodium, calculés en tant que $Na_2O$, et
moins de 1 % en poids de composés oxygénés de nickel, calculés en tant que NiO,
et le corps moulé catalytique présente la forme d'une tablette, ayant un diamètre dans la plage allant de 1,1 à 3,5 mm et une hauteur dans la plage allant de 1,1 à 3,5 mm.

**2.** Procédé selon la revendication 1, **caractérisé en ce que** le corps moulé catalytique présente la forme d'une tablette, ayant un diamètre dans la plage allant de 1,2 à 3,2 mm et une hauteur dans la plage allant de 1,2 à 3,2 mm.

**3.** Procédé selon l'une quelconque des revendications précédentes, **caractérisé en ce que** la masse catalytiquement active du catalyseur contient, avant sa réduction avec de l'hydrogène, moins de 1 % en poids de composés oxygénés de cobalt, calculés en tant que CoO.

**4.** Procédé selon l'une quelconque des revendications précédentes, **caractérisé en ce que** la masse catalytiquement active du catalyseur contient, avant sa réduction avec de l'hydrogène,
25 à 65 % en poids d'oxyde d'aluminium et
30 à 70 % en poids de composés oxygénés de cuivre, calculés en tant que CuO.

**5.** Procédé selon l'une quelconque des revendications précédentes, **caractérisé en ce que** la masse catalytiquement active du catalyseur contient, avant sa réduction avec de l'hydrogène, 0,05 à 1 % en poids de composés oxygénés de sodium, calculés en tant que $Na_2O$.

**6.** Procédé selon l'une quelconque des revendications précédentes, **caractérisé en ce que** la masse catalytiquement active du catalyseur ne contient pas de nickel, de cobalt et/ou de ruthénium.

**7.** Procédé selon l'une quelconque des revendications précédentes, **caractérisé en ce que** la réaction a lieu de manière isotherme, avec une déviation de température d'au plus $\pm$ 8 °C.

**8.** Procédé selon l'une quelconque des revendications précédentes, **caractérisé en ce que** la réaction a lieu dans un réacteur tubulaire.

**9.** Procédé selon la revendication précédente, **caractérisé en ce que** la réaction a lieu dans le réacteur tubulaire selon un mode à gaz circulaire.

**10.** Procédé selon la revendication précédente, **caractérisé en ce que** la quantité de gaz circulaire se situe dans la plage allant de 40 à 1 500 $m^3$ (à la pression d'exploitation)/[$m^3$ de catalyseur (volume apparent) ·h].

**11.** Procédé selon l'une quelconque des revendications précédentes, **caractérisé en ce que** la réaction a lieu dans un réacteur à faisceau de tubes ou dans une unité à gaine unique.

**12.** Procédé selon l'une quelconque des revendications précédentes, **caractérisé en ce que** la réaction est réalisée à une pression absolue dans la plage allant de 1 à 300 bar.

**13.** Procédé selon l'une quelconque des revendications 8 à 12, **caractérisé en ce que** le conditionnement du tube de réacteur/des tubes de réacteur a lieu depuis l'extérieur avec un courant d'huile ou une masse fondue saline.

**14.** Procédé selon l'une quelconque des revendications 9 à 13, **caractérisé en ce que** le gaz circulaire contient au moins 10 % en volume d'hydrogène ($H_2$).

**15.** Procédé selon l'une quelconque des revendications précédentes, **caractérisé en ce que** le composé azoté est utilisé en une quantité molaire de 0,90 à 100 fois par rapport à l'alcool, l'aldéhyde et/ou la cétone utilisés.

**16.** Procédé selon l'une quelconque des revendications précédentes, **caractérisé en ce que** le catalyseur est agencé dans le réacteur sous la forme d'un lit fixe.

**17.** Procédé selon l'une quelconque des revendications précédentes, **caractérisé en ce que** l'alcool, l'aldéhyde et/ou la cétone sont utilisés sous la forme d'une solution aqueuse.

**18.** Procédé selon l'une quelconque des revendications précédentes, **caractérisé en ce que** l'ammoniac, l'amine primaire ou secondaire sont utilisés sous la forme d'une solution aqueuse.

**19.** Procédé selon l'une quelconque des revendications précédentes pour la fabrication de morpholine et/ou d'éther 2,2'-dimorpholinodiéthylique par mise en réaction de diéthylène glycol (DEG) avec de l'ammoniac.

**20.** Procédé selon l'une quelconque des revendications 1 à 18 pour la fabrication de N,N-di(alkyle en $C_{1-4}$)cyclohexylamine par mise en réaction de cyclohexanone et/ou de cyclohexanol avec une di(alkyle en $C_{1-4}$)amine.

**21.** Procédé selon l'une quelconque des revendications 1 à 18 pour la fabrication de pipéridine par mise en réaction de 1,5-pentanediol avec de l'ammoniac.

**22.** Procédé selon l'une quelconque des revendications 1 à 18 pour la fabrication de pyrrolidine par mise en réaction de 1,4-butanediol avec de l'ammoniac.

**23.** Procédé selon l'une quelconque des revendications 1 à 18 pour la fabrication d'hexaméthylène-imine par mise en réaction de 1,6-hexanediol avec de l'ammoniac.

**24.** Procédé selon l'une quelconque des revendications 1 à 18 pour la fabrication de N-(alkyle en $C_{1-4}$)morpholine par mise en réaction de diéthylène glycol avec une mono(alkyle en $C_{1-4}$)amine.

**25.** Procédé selon la revendication précédente pour la fabrication de N-éthyl-morpholine.

**26.** Procédé selon l'une quelconque des revendications 1 à 18 pour la fabrication de N-(alkyle en $C_{1-4}$)-pipéridine par mise en réaction de 1,5-pentanediol avec une mono(alkyle en $C_{1-4}$)amine.

**27.** Procédé selon l'une quelconque des revendications 1 à 18 pour la fabrication de N,N-diméthyléthylamine par mise en réaction d'éthanol avec de la diméthylamine.

**28.** Procédé selon l'une quelconque des revendications 1 à 18 pour la fabrication de N,N-diméthyl-N-propylamine par mise en réaction de n-propanol et/ou de propanal avec de la diméthylamine (DMA).

**29.** Procédé selon l'une quelconque des revendications 1 à 18 pour la fabrication de N,N-diméthyl-N-isopropylamine par mise en réaction d'i-propanol et/ou d'acétone avec de la DMA.

**30.** Procédé selon l'une quelconque des revendications 1 à 18 pour la fabrication de N,N-diméthyl-N-(n-butyl)amine par mise en réaction de 1-butanol et/ou de butanal avec de la DMA.

**31.** Procédé selon l'une quelconque des revendications 1 à 18 pour la fabrication de N,N-diméthyl-N-(iso-butyl)amine par mise en réaction d'i-butanol et/ou d'i-butanal avec de la DMA.

**32.** Procédé selon l'une quelconque des revendications 1 à 18 pour la fabrication de N,N-diméthyl-N-(2-butyl)amine par mise en réaction de 2-butanol et/ou de butanone avec de la DMA.

**33.** Procédé selon l'une quelconque des revendications 1 à 18 pour la fabrication d'éthylpropylamine par mise en réaction de n-propanol et/ou de propanal avec de la monoéthylamine.

**34.** Procédé selon l'une quelconque des revendications 1 à 18 pour la fabrication de 2-(2-di (alkyle en $C_{1-4}$) aminoéthoxy) éthanol et/ou d'éther bis (2-di (alkyle en $C_{1-4}$)aminoéthylique) par mise en réaction de diéthylène glycol avec une di(alkyle en $C_{14}$) amine.

**35.** Procédé selon l'une quelconque des revendications 1 à 18 pour la fabrication de pipérazine par mise en réaction d'aminoéthyléthanolamine (AEEA) avec de l'ammoniac.

**36.** Procédé selon l'une quelconque des revendications 1 à 18 pour la fabrication de 1,2-éthylène-diamine (EDA), de diéthylène-triamine (DETA) et/ou de pipérazine (PIP) par mise en réaction de monoéthylène glycol (MEG) avec de l'ammoniac.

**37.** Procédé selon l'une quelconque des revendications 1 à 18 pour la fabrication de 1,8-diamino-3,6-dioxa-octane et/ou de 1-amino-8-hydroxy-3,6-dioxa-octane par mise en réaction de triéthylène glycol (TEG) avec de l'ammoniac.

**38.** Procédé selon l'une quelconque des revendications 1 à 18 pour la fabrication de 1-méthoxy-2-propylamine (1-méthoxy-isopropylamine, MOIPA) par mise en réaction de 1-méthoxy-2-propanol avec de l'ammoniac.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3275554 A
- DE 2125039 A
- DE 3611230 A
- EP 257443 A
- EP 227904 A
- US 4910304 A
- EP 137478 A
- EP 235651 A1
- EP 816350 A
- US 4739051 A
- EP 514692 A2
- EP 1020455 A
- EP 1106600 A
- US 4806690 A
- DE 19859776 A
- EP 440829 A1
- DE 2445303 A
- EP 440829 A
- WO 07036496 A1
- WO 07036498 A1
- WO 07093514 A1
- WO 07093552 A1
- WO 05110969 A1
- EP 599180 A
- EP 673918 A
- EP 1312600 A
- EP 1312599 A


### In der Beschreibung aufgeführte Nicht-Patentliteratur


- **A. B. STILES.** Catalyst Manufacture - Laboratory and Commercial Preparations. Marcel Dekker, 1983
- Ullmann's Encyclopedia of Industrial Chemistry. vol. B 4, 199-238